# EUROPEAN PATENT APPLICATION

(11) **EP 4 555 970 A1**
(43) Date of publication of application: **21.05.2025**
(21) Application number: 23210478.6
(22) Date of filing: 17.11.2023
(51) Int. Cl.: A61C 5/68, A61J 1/03, A61J 1/20, A61K 6/20, A61K 38/00, A61P 1/02, B65D 75/36, C07K 17/02

(54) **BLISTER PACKAGING FOR PEPTIDES**

(71) Applicant: vVardis AG, 6300 Zug (CH)
(72) Inventor: Abivardi Signer, Golnar, 6340 Baar (CH); Abivardi Brönner, Haleh, 6340 Baar (CH)
(74) Representative: Moré, Solveig Helga

(57) **Abstract**

The present invention relates to an improved packaging for peptides that need to be dissolved before application, for example, self-assembling peptides such as P11-4. In particular, the present invention provides a blister packaging that can be both conveniently used and more cheaply produced than previous packaging, as well as use thereof in application of the peptide, e.g., in treatment of subsurface caries lesions.

## Description

The present invention relates to an improved packaging for peptides that need to be stored in dry form and dissolved before application, for example, self-assembling peptides such as P11-4. In particular, the present invention provides a blister packaging that can be both conveniently used and more cheaply produced than previous packaging, as well as use thereof in application of the peptide, e.g., in treatment of caries lesions.

Tooth decay, also known as dental caries, is one of the most ubiquitous bacterial infections in the world. It is a breakdown of tooth material due to bacterial metabolites, mainly acids made by bacteria when they break down food debris or sugar on the surface or in the biofilm of the teeth. This leads to an imbalance between demineralisation and remineralisation processes. Hard tooth structures, i.e., enamel, dentin and cementum, are damaged by ongoing demineralisation, which results in carious lesions and eventually in the appearance of caries cavities. The earliest sign of a new carious lesion is the appearance of a chalky white spot on the surface of the tooth, a so-called white spot lesion (also designated an incipient carious lesion), i.e. a subsurface lesion. As the demineralisation progresses, the mineralised surface of the lesion (partially) collapses and breaks and a microcavity or a cavity, a hole in the tooth, appears. This is referred to as a (partially) cavitated carious lesion or cavitated carious lesion.

Caries preventative approaches including topical fluoride therapy and application of dental sealants, also known as pit and fissure sealants, have been described. Non-invasive approaches have been suggested for non-cavitated carious lesions, i.e. subsurface lesions. For example, remineralisation is attempted by the application of topical fluoride.

Furthermore, enamel matrix derivatives or self-assembling peptides have been shown to be effective in the remineralisation of subsurface carious lesions (Ruan et al., 2013. Acta Bioma-ter.9(7):7289-97; Ruan et al., 2014. J Vis Exp. 10(89), doi 10.379151606; Schmidlin et al., 2016, J Appl Oral Sci. 24(1), 31-36; Alkilzy et al., 2018, Adv Dent Res. 29(1), 42-47; Brunton et al., 2013, Br Dent J, 215:E6; Kind et al., 2017, J Dent Res., 96(7), 790-797; Kirkham et al., 2007, J Dent Res., 86, 426-430). In WO 2014/027012 A1, a method for the targeted delivery of a self-assembling peptide to a subsurface tooth lesion with the aim of remineralising carious lesions is described. Alkilzy et al. (2018, above) found an additional effect of the application of P11-4, a self-assembling peptide, to early carious lesions, in combination with fluoride, compared to the application of fluoride alone. Further, WO2021/005153 A1 teaches prevention and treatment of cavitated caries lesions with self-assembling peptides.

Amelogenin-based biomineralisation approaches for tooth repair have been described in the context of dentinal hypersensitivity, whitening or bleaching of teeth, and the treatment of caries in WO 2017/123986 A1, US 2014/0186273 A1 and US 2017/0007737 A1.

In WO 2017/168183 A1, a biomimetic mineralised apatite structure based on elastin-like peptides is described for use as dental restorative material, in particular for the reconstruction of enamel, and for use in the treatment of dental diseases such as dental caries.

Furthermore, casein-based strategies have been developed for remineralisation of enamel and prevention and/or treatment of caries or tooth erosion (e.g. WO 00/06108 A1 and WO 2010/042754 A2). Casein phosphopeptide amorphous calcium phosphate complexes (CPP-ACP) and CPP-stabilised amorphous calcium fluoride phosphate complexes have been described in combination with glass-ionomer cement as remineralising dental restorative materials, e.g., in WO 02/094204 A1. Some studies have also shown that caries progression is slowed or arrested under sealants when applied to non-cavitated carious lesions (Griffin S.O. et al., 2008, J Dent Res., 87(2), 169-174).

Thus, peptides may play an important role in prevention and treatment of caries. In particular, self-assembling peptides such as P11-4 are already successfully used for treatment of subsurface caries lesions. However, so far, more wide-spread use is hampered by the costs of treatment. The use of self-assembling peptides in treatment of caries lesions is rendered more difficult by the fact that the peptide should to be monomeric, in particular, for treatment of subsurface lesions, because assembled peptides cannot diffuse into the enamel and contribute to thorough remineralisation. In the presence of water, the self-assembling peptides tend to assemble. Assembly can be controlled by pH adaptation, but in clinical practice, adapting the pH before application, as e.g.,done by Alkilzy et al. (above) is impractical. Therefore, on the basis of WO 2014/027012 A1, a method for producing a dry composition ready for treatment of a caries lesion upon resuspension was developed. Currently, a sponge coated with a peptide composition produced according to said method is clinically used in an applicator with a separate chamber comprising a liquid (Curodont Repair, Credentis AG, Switzerland), and for application, the liquid from said separate chamber is used to resuspend the peptide. It can then be administered with the sponge. However, production of these applicators is expensive, a lot of plastic waste is produced, and if a plurality of lesions are to be treated, it is not very comfortable to use.

It is therefore the object of the invention to at least partially overcome drawbacks mentioned above and to provide a packaging which is easy and comfortable to handle and which helps to make the day-to-day business of dentist's offices more efficient.

This object is achieved by the present invention, in particular, by the claimed subject-matter.

The invention provides a blister packaging comprising a blister sheet with at least one cavity and a cover sheet laid over and bonded to the blister sheet surrounding the at least one cavity, wherein the at least one cavity of the blister comprises a poriferous support coated with a peptide.

This configuration allows for an easy handling of the supports that is already coated with an adequate dose of peptide sufficient for the treatment of at least one lesion. The support can be safely stored, in the absence of water. Directly before treatment, it can be, e.g., as provided herein, contacted with a desired amount of a liquid such as a buffer or, preferably, sterile water. This leads to resuspension of the peptide, which can then be applied to a caries lesion.

For treatment of caries lesions, as explained above and known in the art, it is beneficial if the peptide is a self-assembling peptide in monomeric form. Assembled (polymeric) peptide can be used for other purposes ,e.g., for treatment of tooth sensitivity or for preventing tooth erosion.

Peptides that may be used in dental treatment, e.g, caries treatment or prevention, are known in the art, and, e.g., mentioned herein. For example, self-assembling peptides (SAP) are known for their excellent characteristics in treatment and prevention of caries lesions (e.g., WO 2004/007532 A1). WO 2004/007532 A1 discloses peptides that are capable of forming three-dimensional scaffolds, thereby promoting nucleation of de-novo calcium phosphate. These artificial peptides assemble in one dimension to form beta-sheet, and higher order assemblies such as tape-like assemblies. Three-dimensional supramolecular structures of SAP can be formed, which have an affinity for/ to calcium phosphate.

In the context of the present invention, self-assembling peptides taught in WO 2004/007532 A1, US10/521,628, US12/729,046, US13/551,878, US 14/062,768, or WO2014/027012 A1, which are all fully incorporated herein by reference, are preferred. In particular, self-assembling peptides having a net charge of +2 or -2 at pH 7.5, may be used in monomeric or assembled form.

Self-assembling peptides used in the present invention preferably comprise a sequence of the formula X1-X2-X1-X2-X1, wherein X1 is an amino acid with an acidic side chain and X2 is an amino acid with a hydrophobic side chain. For example, the self-assembling peptide used in the present invention comprises the consensus sequence SEQ ID NO: 1, X1-X2-X1-X2-X1, wherein X1 is independently selected from the group consisting of glutamic acid, aspartic acid, glutamine and ornithine, and X2 is independently selected from the group consisting of alanine, valine, isoleucine, leucine, methionine, phenylalanine, tyrosine, tryptophan and glutamine, or an amino acid sequence having at least 80% identity thereto. Independently selected means that, e.g., X1 in positions 1, 3 or 5 of the sequence above can be different from each other. Of course, they can also be identical.

Preferably, self-assembling peptides used in the invention comprise SEQ ID NO: 2, X1-X2-X1-X2-X1, wherein X1 is independently selected from the group consisting of glutamic acid and ornithine, and X2 is independently selected from the group consisting of tryptophan and phenylalanine.

In a further embodiment, self-assembling peptides used in the invention may comprise SEQ ID NO: 3, X3-F-X1-W-X1-F-X1, wherein X1 is independently selected from the group consisting of glutamic acid and ornithine, and X3 is selected from the group consisting of arginine, glutamic acid and ornithine, wherein X3 preferably is arginine.

Furthermore, self-assembling peptides used in the invention may comprise SEQ ID NO: 4 or, preferably, consist thereof: X4-X4-X3-F-X1-W-X1-F-X1-X4-X4, wherein X1 is independently selected from the group consisting of glutamic acid and ornithine, and wherein X3 is selected from the group consisting of arginine, glutamic acid and ornithine, and wherein X4 is independently selected from the group consisting of glutamine, glutamic acid, serine, threonine and ornithine. X3 preferably is arginine. Independently, X4 preferably is glutamine.

Self-assembling peptides of the invention may comprise SEQ ID NO: 5, or, preferably, consist thereof: Q-Q-R-F-X1-W-X1-F-X1-Q-Q, wherein X1 is independently selected from the group consisting of glutamic acid and ornithine.

Preferably, the self-assembling peptides used in the present invention comprise or consist of a sequence selected from the consensus sequences listed in Table 1 below.

Most preferably, said peptides comprise the specific peptides listed in Table 2 or consist thereof. Of course, self-assembling peptides assembling in combination with another self-assembling peptide, e.g., as disclosed herein, may be formulated in one kit or in one composition.

Peptides of SEQ ID NO: 6, 9, 11, 12, 16 or 17 are particularly advantageous, e.g., as they can be used in relatively low concentrations, they are highly compatible with cells and have beneficial charge distribution.

Preferably, the self-assembling peptide comprises the sequence of SEQ ID NO: 6 or consists thereof. A peptide consisting of a sequence of SEQ ID NO: 6 is also designated P11-4, and is preferred throughout the invention. In another preferred embodiment, the self-assembling peptide comprises the sequence of SEQ ID NO: 9 or consists thereof (P11-8).

The kit or composition of the invention may also comprise at least one self-assembling peptide having at least 45% sequence identity to a peptide consisting of SEQ ID NO: 6. Preferably, the peptide has at least 54%, at least 63%, at least 72%, at least 81% or at least 90% sequence identity to a peptide consisting of SEQ ID NO: 6, or is said peptide. Peptides of the invention are, for example, 11 amino acids in length.

Self-assembling peptides may be modified peptides comprising an Ac-N-terminus and/or NH₂-C-Terminus, preferably, both, or non-modified peptides. As non-blocked forms tend to start a deaminization reaction, the termini of all self-assembling peptides of SEQ ID NO: 1 are preferably blocked to increase stability. In particular, peptides of SEQ ID NO: 6, 9, 11, 12, 16 and 17 may comprise an Ac-N-terminus and NH₂-C-Terminus. SEQ ID NO: 18-29 correspond to modified peptides of the invention.

**Table 1: Consensus sequences of preferred self-assembling peptides**

| **SEQ ID NO** | **Peptide name** | **Sequence** | **Exemplary SAP** |
|---|---|---|---|
| SEQ ID NO: 1 | Consensus sequence 1 | X1-X2-X1-X2-X1, | P11-2, P11-4, P11-5, P11-8, P11-12, P11-13, P11-14, P11-17, P11-19, P11-20, P11-28, P11-29 |
| | | wherein X1 is independently selected from the group consisting of glutamic acid, aspartic acid, glutamine and ornithine, and X2 is independently selected from the group consisting of alanine, valine, isoleucine, leucine, methionine, phenylalanine, tyrosine, tryptophan and glutamine | |
| SEQ ID NO: 2 | Consensus sequence 2 | X1-X2-X1-X2-X1, | P11-4, P11-8, P11-12, P11-13, P11-14, P11-17, P11-28, P11-29 |
| | | wherein X1 is independently selected from the group consisting of glutamic acid and ornithine, | |
| | | and X2 is independently selected from the group consisting of tryptophan and phenylalanine | |
| SEQ ID NO: 3 | Consensus sequence 3 | X3-F-X1-W-X1-F-X1, wherein X1 is independently selected from the group consisting of glutamic acid and ornithine, | P11-4, P11-8, P11-12, P11-13, P11-14, P11-17, P11-28, P11-29 |
| | | and X3 is selected from the group consisting of arginine, glutamic acid and ornithine, wherein X3 preferably is arginine | |
| SEQ ID NO: 4 | Consensus sequence 4 | X4-X4-X3-F-X1 -W-X1 -F-X1 -X4-X4, wherein X1 is independently selected from the group consisting of glutamic acid and ornithine, | P11-4, P11-8, P11-12, P11-13, P11-14, P11-17, P11-28, P11-29 |
| | | and wherein X3 is selected from the group consisting of arginine, glutamic acid and ornithine, | |
| | | and wherein X4 is independently selected from the group consisting of glutamine, glutamic acid, serine, threonine and ornithine. X3 preferably is arginine. Independently, X4 preferably is glutamine. | |
| SEQ ID NO: 5 | Consensus sequence 5 | Q-Q-R-F-X1-W-X1-F-X1-Q-Q, wherein X1 is independently selected from the group consisting of glutamic acid and ornithine. | P11-4, P11-8 |

**Table 2: Preferred self-assembling peptides. Positions X1 are underlined**

| **SEQ ID NO** | **Peptide name** | **Sequence (One letter code)** | **% amino acid identity** to **P11-4** (ClustalW (2.1, standard parameters)) |
|---|---|---|---|
| SEQ ID NO: 6 | P11-4 | QQRFEWEFEQQ | 100 |
| SEQ ID NO: 7 | P11-2 | QQRFQWQFEQQ | 81.8 |
| SEQ ID NO: 8 | P11-5 | QQRFOWOFQQQ | 72.7 |
| SEQ ID NO: 9 | P11-8 | QQRFOWOFEQQ | 81.8 |
| SEQ ID NO: 10 | P11-12 | SSRFOWOFESS | 45.4 |
| SEQ ID NO: 11 | P11-13 | EQEFEWEFEQE | 72.7 |
| SEQ ID NO: 12 | P11-14 | QQOFOWOFOQQ | 63.6 |
| SEQ ID NO: 13 | P11-17 | TTRFEWEFETT | 63.6 |
| SEQ ID NO: 14 | P11-19 | QQRQOQOQEQQ | 54.5 |
| SEQ ID NO: 15 | P11-20 | QQRQEQEQEQQ | 72.7 |
| SEQ ID NO: 16 | P11-28 | OQOFOWOFOQO | 45.4 |
| SEQ ID NO: 17 | P11-29 | QQEFEWEFEQQ | 90.9 |
| SEQ ID NO: 30 | P11-16 | NNRFOWOFENN | 45.4 |
| SEQ ID NO: 31 | P11-18 | TTRFOWOFETT | 45.4 |
| SEQ ID NO: 32 | P11-26 | QQOQOQOQOQQ | 36.4 |
| SEQ ID NO: 33 | P11-31 | SSOFOWOFOSS | 27.3 |

The self-assembling peptides preferably do not have restriction sites for the subject's endopeptidases. They also do not need to comprise a special recognition motif for cells.

In a preferred embodiment, the peptide is a P11-4 self-assembling peptide having SEQ ID NO: 6.

The peptide in monomeric form is preferably prepared as disclosed in WO 2014/027012 A1, so that it can be directly used after being resuspended in water. In the context of the invention, the resuspended peptide solution can be transferred from the packaging to the tooth surface using the poriferous support. The support can, e.g., be held with a pinzer, and the tooth surface contacted with the support. The tooth surface is advantageously pretreated to remove plaque and biofilm, e.g.,as dislosed by Alkilzy et al (above) or in the instructions for use of Curodont Repair.

The amount of peptide coated on the support may vary, e.g., depending on the number of cavities for treatment of which it is intended. For example, 0.1 to 10 mg of peptide (preferably, self-assembling peptide,e.g., P11-4) may be coated on one support, such as 0.5 to 5 mg, preferably, 1-2 mg or 1.4 to 1.6 mg.

In an advantageous embodiment the blister packaging comprises a plurality of cavities comprising a poriferous support coated with a peptide. Such cavities are designated first cavities. In this way the poriferous supports can be packaged by known in the art packaging machines in large quantities, which reduces costs of the inventive blister package.

Further, the blister packaging might comprise at least one second cavity, optionally, a plurality of second cavities comprising a liquid, wherein such cavities are designated second cavities. Therewith, the already dosed liquid for activating the peptide coating can be provided by the same blister packaging. This reduces preparation workings for the poriferous peptide coated support, as both components for the treatment of the tooth surfaces can be provided simultaneously. The liquid is a water-based liquid. It can be a buffer, but, preferably, it is water, such as sterile water. The amount of liquid is chosen based on the amount of peptide on the support, e.g., to yield a peptide concentration of about 0.1-100 mg/mL, such as 1-50 mg/mL, preferably, 5-20 mg/mL or about 10 mg/mL. For example, the volume of the liquid can be 10µL to 1mL, e.g.,16µL to 500 µL, 50 µL to 200 µL or 100 to 150 µL, preferably, about 100 µL.

In one embodiment of the blister packaging, adjacent to each first cavity, a second cavity is arranged. Optionally, each blister packaging only comprises one first and one second cavity, but it may also comprise a plurality of first and second cavities.

The packaging solves therefore in reliable manner the problem of conveniently handling and providing two components simultaneously, which otherwise may have to be prepared or pH adapted shortly before a treatment or provided in a complicated applicator. This reduces the preparation workings of professional dental stuff or cost, and is beneficial for the day-to-day business at a dentist's offices.

The bonding of the cover sheet to the blister sheet in the blister packaging of the invention maintains in dry environment in the cavities of the blister that comprise the peptide-coated poriferous supports. The bonding is thus airtight and water-tight. This can be carried out by hot sealing or gluing the cover sheet onto the blister sheet which contributes to a sterile airtight enclosure of the coated support.

The cover sheet may be firmly attached to the blister sheet, e.g., to provide a push-through pack. A push-through pack comprises a cover sheet that is brittle, making it possible to press the support out while breaking the cover sheet, and/or by a semi-rigid formed cavity being sufficiently collapsible to be able to dispense the poriferous support by means of pressing it out with the thumb. Additionally, breaking the cover sheet with an object like a tweezer or a fingernail may make the pressing out easier.

Alternatively, and preferably, the cover sheet may be removably attached to the blister sheet. Removable attached in the context of the invention means that it is easily possible to remove the cover sheet from the blister sheet by hand and without undue force. In particular, the force should not lead to shaking of the packaging that may lead to the support falling out. Preferably, removal is possible with one hand. A flap of the cover sheet, e.g., at the edge of the blister packaging may be loose to simplify holding and removing the sheet. Thus, the user can easily access the support lying in the cavity.

Further, the cover sheet may be removable attached, as described herein, and the blister packaging comprises a plurality of cavities. In this case, preferably, the cover sheet may be individually removable for each cavity comprising a poriferous support coated with a peptide. If second cavities comprising a liquid are present, the cover sheet over these may, optionally, similarly be individually removable.

Optionally, the cover sheet is individually removable for each pair of first and second cavities together, i.e., the cover sheet for both first and second cavity of an adjacent pair of cavities is removable together without disturbing the cover sheet over other cavities. This enables a comfortable handling of the respective cavities.

To allow for individually removing (or individually removing together) certain regions of cover sheet, the cover sheet, when bonded to the blister sheet, may comprise a perforation, which enables the user to individually remove the cover sheet from a certain cavity or pair of cavities. Alternatively, the cover sheet of each individual cavity may be individualized during or after the bonding of the cover sheet.

The cover sheet of each individual cavity may additionally comprise a section which is not bonded with the blister sheet that enables the user to grip the not bonded flap of the cover sheet in order to tear of the latter from the blister sheet.

Additionally, or alternatively, the blister sheet may comprise predetermined breaking lines, which enables the user to individualize single cavities or pairs of first and second cavities from the blister sheet according to the actual needs during the treatment. In this case the cavity is still closed during breaking. Before use, only the cover sheet over the desired part of the sheet is removed so that the rest of the packaged support and/or liquid remains sterile during the handling of the individual cavity.

These features contribute to a quick and easy handling of the poriferous support and minimizes the preparation workings for dental treatments requiring a sterile poriferous support to a minimum.

In one embodiment, the second cavities are configured to, at least partly, preferably, completely, receive the support so that the support can absorb the liquid within the second cavity. In this manner, a container is provided, in which the support can absorb and, if required, briefly rest during its preparation. More preferably, the first and second cavities are dimensioned equally. This ensures that the support can be received by the second cavity and this provision also lowers the production costs of the blister sheet, as the blister sheet can be uniformly produced.

In another embodiment, the packaging is configured such that the removal of the cover sheet also removes a barrier between the first and the second cavity of the pair and allows for the liquid contacting the peptide coated support. Therewith, the liquid of the second cavity is able to flow into the first cavity holding the peptide coated support, e.g., when inclining the blister packaging towards the first cavity. This also results in a comfortable and fast preparation of the peptide coated support without handling of the latter.

Throughout the invention, the poriferous support can be, e.g., a sponge, a swab, a fleece or wool, e.g., cotton wool. The poriferous support is not explicitly limited to the before named examples. The poriferous support may also be a tampon, a plug, a dapper or any other pharmaceutically acceptible liquid absorbing product for medical use.

Medical sponges, swabs and cotton wools are well known in the art. They are individualizable in size which can be mechanized manufactured at low costs and in great quantity.

The poriferous support may e.g., comprise or consist of polyvinyl alcohol (PVA), cotton wool, polyethylene (PE), polyethylene terephthalate (PET), polypropylene (PP), or cyclic olefin copolymers (COC). These listed materials are widely used and well suited for the absorption of liquids when processed to nonwovens or foams. The poriferous support may also be configured to comprise one or more materials listed above so that optimal absorption characteristics can be achieved and medical requirements can be fulfilled.

Preferably, the peptide is coated on the poriferous support together with an excipient, optionally, together with a cellulose-based excipient that improves binding to the support. Preferably, carboxymethylcellulose may be used as excipient. Coating the poriferous support together with the excipient results in more reliable results, and the coating can be uniformly applied to the poriferous support.

The peptide may coated on the poriferous support by a method selected from spray drying, fluidized bed coating, dip coating or mixing. These methods lead to reliable reproductive results, whereas the coating can be uniformly applied to the poriferous support.

Preferably, the blister sheet and/or cover sheet comprise polyethylene (PE), polyethylene terephthalate (PET), polypropylene (PP), cyclic olefin copolymers (COC), polyurethane (PU), polystyrene (PS), or polyvinyl chloride (PVC), and/or a metal foil, preferably an aluminium foil or a plastic-laminated metal foil therefrom. These materials ensure that the concentration of the peptide coating remains constant when stored within the cavity, and prevents contact of the support with water which further contributes to an optimal dental treatment as originally intended.

Of note it is not required that the liquid, e.g., water, is also stored in the blister packaging. It should however be preferably provided together in a kit with the blister packaging of the invention, advantageously, in a form that allows easily measuring out and adding the desired amount of liquid. Thus, in another aspect, the invention provides a kit that in addition to the blister packaging comprises a container comprising a sterile liquid, preferably water. Optionally, the container allows for providing a measured amount of said liquid to the cavity comprising a poriferous support. The container may be a multi-dose container, but it preferably is an ampoule, e.g, a break-off ampoule containing one dose of the liquid, (e.g., as described above, such as 100 µL) which can then be dispensed into the cavity comprising the poriferous support.

It is particularly preferred to use the blister packaging or the kit in treatment of a caries lesion, optionally, a non-cavitated (or subsurface) caries lesion. As described in the art and discussed above, treatment of cavitated caries lesions is also possible, e.g., for preventing secondary caries. The peptide can also be applied, e.g., to a bone lesion.

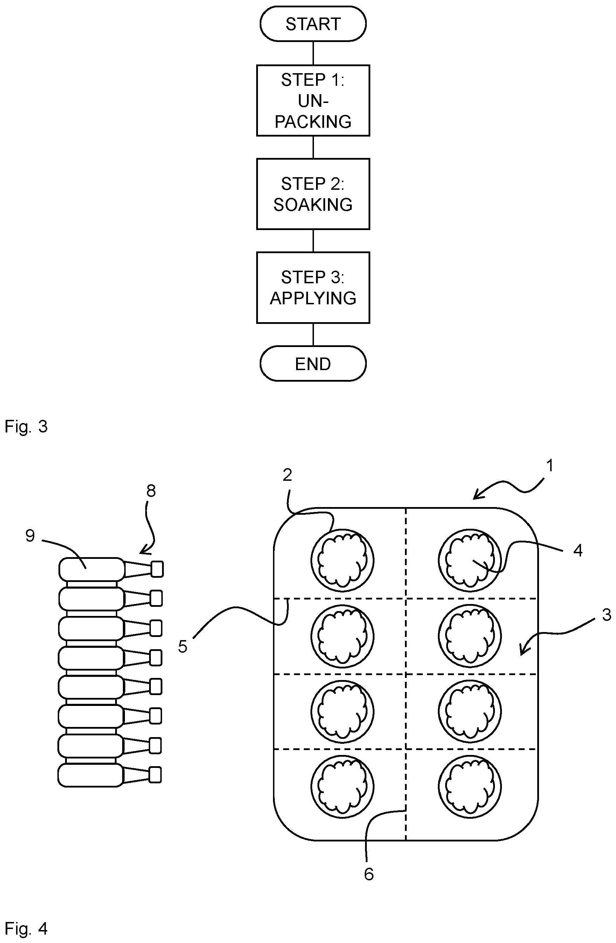

The invention also provides a method of treating a tooth, e.g., a subsurface caries lesion, using the packaging and support as provided herein, e.g., comprising steps as laid out below, in particular, in Fig. 3.

All references cited herein are herewith fully incorporated.

Without intending to limit the invention, preferred embodiments and advantages of the blister packaging and the kit according to the invention will now be described with reference to the attached drawings in which:
Fig. 1 is a top view on the blister packaging according to a first embodiment;
Fig. 2 is a top view on the blister packaging according to a second embodiment;
Fig. 3 is a schematic process flow describing the method of preparation of the coated poriferous support; and
Fig. 4 is a top view of the blister packaging according to a third embodiment.
   1. Blister packaging
   2. Cavity
   3. Cover seal
   4. Support
   5. Transversal perforation
   6. Longitudinal perforation
   7. Liquid
   8. Liquid container
   9. Single-use-ampoule

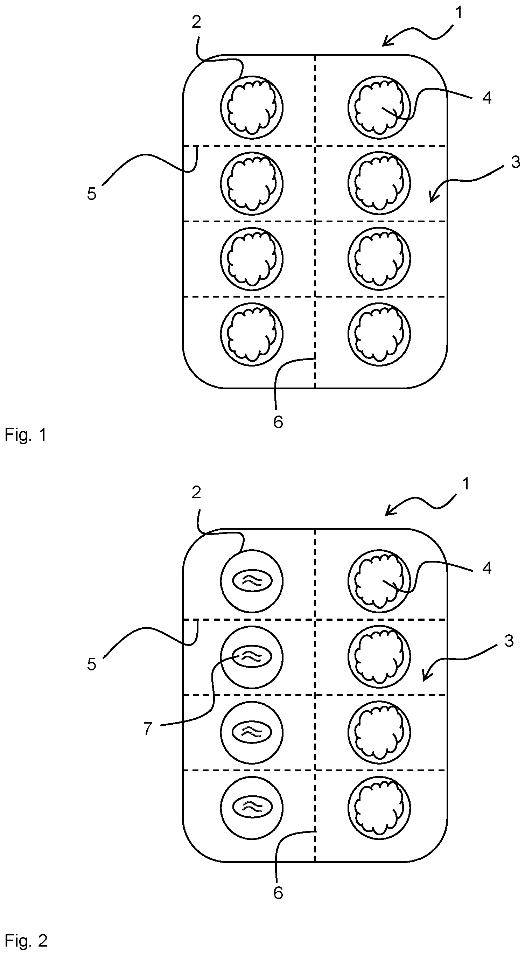

Fig. 1 shows a first preferred embodiment of the blister packaging 1 according to the invention. In this embodiment, the blister packaging 1 comprises eight cavities 2 each comprising a poriferous support 4 coated with a peptide. The cover sheet 3 is bonded to the blister sheet by thermal sealing. The cover sheet is in this embodiment made of aluminium, whereas the blister sheet is made of a non-peptide absorbing plastics such as polyethylene (PE), but is not limited thereto. Other suited above-mentioned non-peptide plastic materials may also be used. In this embodiment the cover sheet 3 is made of a transparent PE plastic, which enables the user to observe the quantity and condition of the poriferous supports 4 within the cavities 2. The cavities 2 are dimensioned in such way that they are able to fully receive the poriferous support 4.

The poriferous support 4 is in this embodiment, e.g, a cotton wool coated with a self-assembling peptide in monomeric form of SEQ ID NO: 6. The peptide coating is sufficiently dry so that the poriferous support 4 has long shelf live. The peptide is coated on the poriferous support together with an excipient or filler, e.g,. by dip coating. Other suitable mentioned coating methods can be used.

In one embodiment, the cover sheet 3 is made of aluminium and is sufficiently rigid so that the blister packaging 1 configured as a push-through pack making it possible to press the poriferous support 4 out while breaking the cover sheet 3. Additionally, breaking the cover sheet 3 with an object like a tweezer makes the pressing out easier. Alternatively, the cover sheet 3 is manually removable, in particular, individually.

Further, the blister packaging shown in Fig. 1 comprises multiple transversal perforations 5 which are configured as predetermined breaking lines to separate at least one or a pair of cavities 2 in a transversal direction from the blister packaging 1. The transversal perforations 5 are arranged between a pair of two cavities 2. The blister packaging 1 of Fig. 1 also comprises a longitudinal perforation 6, which is configured as a predetermined breaking line to separate at least one cavity 2 in the longitudinal direction from the blister packaging 1.

The embodiment is not limited to the quantity of cavities 2 or poriferous supports 4. Other not shown embodiments might comprise more or less cavities 2 and poriferous supports 4 as well as transversal and longitudinal perforations 5, 6.

The shown second embodiment of the inventive blister packaging 1 in Fig. 2 is structurally equal to the first embodiment described above. The difference between the first and second embodiment lies in that the cavities 2 on the right side of the blister packaging 1, the first cavities 2, comprise the poriferous support 4 and the cavities 2 on the left side of the blister packaging 1, the second cavities 2, comprise a liquid 7 to be soaked into the poriferous support 4. In particular, the liquid 7 is sterile water sufficiently dosed relative to the peptide coating of the poriferous support 4.

The second cavities 2 are adjacent arranged to the first cavities 2 and equally sized so that they can fully receive the poriferous support 4. A first cavity 2 and a second cavity 2 forms a pair. Further, the first and second cavities a configured in such way, that they can rest on a plane. With this configuration, when the cavities 2, especially the second cavity 2 is opened, the cavity 2 can be used as a container holding the poriferous support 4 which can soak the liquid 7 comprised in the second cavity 2.

A third embodiment of the inventive blister packaging 1 is disclosed in Fig. 4. The blister packaging 1 of the third embodiment is structurally identical to that already described in the first embodiment of Fig. 1. The third embodiment illustrates an inventive kit comprising a separate sterile liquid container 8, that provides a measured amount of said liquid, in particular sterile water. In this embodiment, the liquid container 8 comprises multiple unit dose packagings 9, more particular single-use ampoules, of about 0.3 mL of sterile water. The unit dose packagings 9 are interconnected with each other which simplifies the handling of the kit. The unit dose packagings 9 can be manually separated by tearing them off by hand or cutting them through at an interconnection section. The interconnection section is configured as a predetermined breaking line.

Fig. 3 discloses a method of preparing the poriferous support 4. The embodiment of the method is carried out by use of the inventive blister packaging 1 according to the second embodiment with reference to Fig. 2.

The first step comprises unpacking of the peptide coated poriferous support 4. The poriferous support 4 can be unpacked by pushing it through the cover layer 3 with the thumb, whereby the cover sheet 3 brakes and releases the poriferous support 4.

The following step comprises soaking the poriferous support 4 within the second cavity 2 holding the sterile water. The poriferous support 4 is inserted into the second cavity 2, which receives it completely. Optionally, the poriferous support 4 may be left to rest until it has absorbed at least partly, preferably fully, the liquid 7 of the second cavity 2. During the soaking step, the second and/or first cavity can be placed on a plane, while the poriferous support 4 soaks the liquid 7.

The final third step comprises applying of the into the poriferous support 4 soaked in liquid 7 onto teeth surfaces of the subject. The poriferous support 4 can be simply handled by tweezers. Slightly pressing the poriferous support 4 with the tweezers or against a tooth surface helps to release the liquid 7.

With the subject matter of the present invention a blister packaging has been provided which comprises a simple design, is easy to manufacture and ensures an enhanced handling and usability when used in the day-to-day business of a dentist's office.

## Claims

1. A blister packaging comprising a blister sheet with at least one cavity and a cover sheet laid over and bonded to the blister sheet surrounding the at least one cavity, wherein the at least one cavity of the blister comprises a poriferous support coated with a peptide.

2. The blister packaging of claim 1, wherein the peptide is a self-assembling peptide in monomeric form.

3. The blister packaging of any of the preceding claims, wherein the peptide is a self-assembling peptide having SEQ ID NO: 1, preferably, wherein the peptide is a self-assembling peptide having SEQ ID NO: 6.

4. The blister packaging of any of the preceding claims, comprises a plurality of cavities comprising a poriferous support coated with a peptide, wherein such cavities are designated first cavities.

5. The blister packaging of any of the preceding claims, further comprising at least one second cavity, optionally, a plurality of second cavities comprising a liquid, preferably, sterile water, wherein such cavities are designated second cavities.

6. The blister packaging of any of the preceding claims, wherein the cover sheet is manually removable, wherein, if the blister packaging comprises several cavities, the cover sheet is individually removable for each cavity comprising a poriferous support coated with a peptide and, if present, optionally, for each cavity comprising a liquid.

7. The blister packaging of any of the preceding claims, wherein adjacent to each first cavity, a second cavity is arranged,
optionally, wherein the cover sheet is individually removable for each pair of first and second cavities together.

8. The blister packaging of claim 7, wherein removal of the cover sheet removes a barrier between the first and the second cavity of the pair and allows for the liquid contacting the peptide-coated support.

9. The blister packaging of any of the preceding claims, wherein blister packaging is a push-through pack.

10. The blister packaging of any of the preceding claims, wherein the poriferous support is a sponge, a swab, a fleece or cotton wool.

11. The blister packaging of any of the preceding claims, wherein the poriferous support comprises polyvinyl alcohol (PVA), cotton wool, polyethylene (PE), polyethylene terephthalate (PET), polypropylene (PP), or cyclic olefin copolymers (COC).

12. The blister packaging of any of the preceding claims, wherein the peptide is coated on the poriferous support together with a filler, optionally, together with carboxymethylcellulose.

13. The blister packaging of any of the preceding claims, wherein the peptide is coated on the poriferous support by a method selected from spray drying, fluidized bed coating, dip coating, or mixing.

14. The blister packaging of any of the preceding claims, wherein the blister packaging of any of the preceding claims, wherein the blister sheet and/or cover sheet comprise polyethylene (PE), polyethylene terephthalate (PET), polypropylene (PP), cyclic olefin copolymers (COC), polyurethane (PU), polystyrene (PS), or polyvinyl chloride (PVC), and/or a metal foil, preferably an aluminium foil or a plastic-laminated metal foil therefrom.

15. A kit comprising the first blister packaging of claim 1 to 4, wherein the kit further comprises a container comprising a sterile liquid, preferably water, optionally, wherein the container allows for providing a measured amount of said liquid to the cavity comprising a poriferous support.

16. The blister packaging of any of claims 1-14 or the kit of claim 15 for use in treatment of a caries lesion, optionally, a non-cavitated caries lesion.
